# EUROPEAN PATENT APPLICATION

(11) **EP 1 847 592 A1**
(43) Date of publication of application: **24.10.2007**
(21) Application number: 06127331.4
(22) Date of filing: 29.12.2006
(51) Int. Cl.: C12M 1/14, C12M 1/18, C12M 3/04

(54) **Cell co-culture apparatus for researching cell interaction**

(30) Priority: 21.04.2006 KR 20060036404
(71) Applicant: Samsung Electronics Co., Ltd., Suwon-si, Gyeonggi-Do (KR)
(72) Inventor: Cheong Kwang-ho Samsung Advanced Institute, Giheung-gu, Yongin-si Gyeonggi-do (KR); Park, Chin-sung c/o Samsung Advanced Institute, Giheung-gu, Yongin-si Gyeonggi-do (KR)
(74) Representative: Zimmer, Franz-Josef

(57) **Abstract**

Provided are a cell co-culture apparatus (1) and a method of co-culturing and isolating cells using the cell co-culture apparatus (1) to research an interaction between the cells by direct contact. The cell co-culture apparatus (1) includes a first substrate (10) having a plurality of projections (12) on the surface (11) of the first substrate (10), wherein the projections (12) have top surfaces (13) for culturing cells; a second substrate (20) having the same number of channels (21) as the number of the projections (12) of the first substrate (10) such that the channels (21) fit with the projections (12) of the first substrate (10); and a third substrate (30) having a plurality of channels (31) corresponding with the top surfaces (13) of the projections (12) of the first substrate (10) and a plurality of channels (21) corresponding with the substrate surface (22) between the channels (21) of the second substrate (20).

## Description

### BACKGROUND OF THE INVENTION

### 1. Field of the Invention

The present invention relates to a cell co-culture apparatus for researching an interaction between cells, and more particularly to a cell co-culture apparatus for researching an interaction between cells by a direct contact.

### 2. Description of the Related Art

The interaction between the cells is very important in growth, migration, and differentiation of cells. Furthermore, tissues serve their functions by the interaction between the cells, and thus, in the case of *in vitro* cell research for treatment, the interaction between the cells is more important. Thus, a means that allows the research on the interaction between the cells by direct contact is required.

At a specific molecular level, the interaction between the cells includes a signal transfer between the cells through a body fluid, such as growth hormones, cytokines, etc. and a signal transfer by a direct contact between the cells.

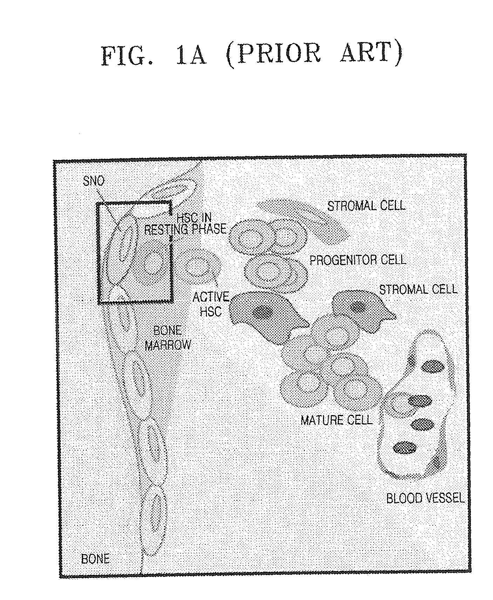

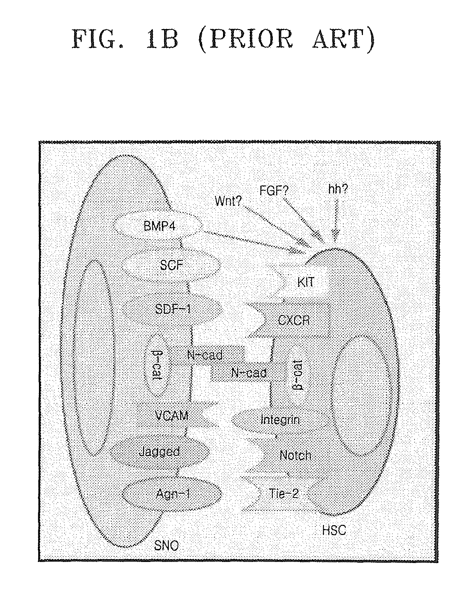

Representative examples of the signal transfer by the direct contact between the cells include an interaction between a hematopoietic stem cell (HSC) and spindle-shaped N-cadherin osteoblast (SNO). The HSC interacts with the SNO via N-cadherin, as illustrated in FIG. 1 B, in order to retain stemness. That is, in order to retain the stemness of the HSC, the SNO must be adjacent to the HSC, as schematically illustrated in FIG. 1A.

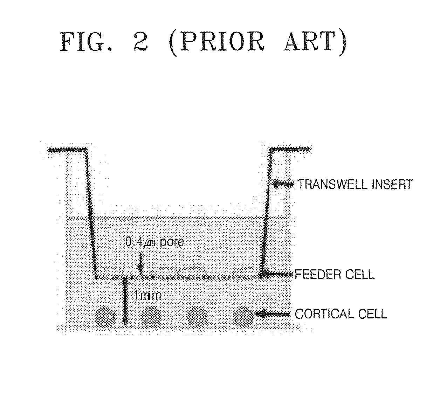

Shen Q. et al. reported that endothelial cells provide circumstances wherein the stemness of neural stem cells are retained using a transwell insert. The research on the interaction between the endothelial cells and the neural stem cells was performed using the transwell insert. Specifically, the transwell insert is used as illustrated in FIG. 2 (Shen Q et al., Endothelial cells stimulate self-renewal and expand neurogenesis of neural stem cells. Science. 2004 May 28; 304(5675):1338-40).

Referring to FIG. 2, two types of cells that are to be investigated for their interaction with each other are separated from each other by the transwell insert as used in the above-mentioned research. Thus, in this case, only the interaction between the cells by a soluble factor through a liquid may be researched. An apparatus for researching the interaction by a direct contact between the cells has not been reported and is required.

### SUMMARY OF THE INVENTION

The present invention provides a cell co-culture apparatus for researching an interaction between cells.

The present invention also provides a method of co-culturing and isolating cells using the above cell co-culture apparatus.

The cell co-culture apparatus is designed for culturing at least two different types of cells that are in contact with each other at the interface between the two different types of cells and isolating one of the different types of cells from the other in order to confirm a change in each of the cells during the contact culture. The cell co-culture apparatus is a set comprising three substrates, wherein the first substrate is for seeding and culturing the first cells among the cells that are to be investigated for the cell interaction, the second substrate is for seeding and culturing the second cells from among the cells that are to be investigated for the cell interaction, and the third substrate is for providing channels through which the first cells and the second cells may easily be seeded on the first substrate and the second substrate.

According to an aspect of the present invention, there is provided a cell co-culture apparatus comprising: a first substrate having a plurality of projections on the surface of the first substrate, wherein the projections have top surfaces for culturing cells; a second substrate having the same number of channels as the number of the projections of the first substrate such that the channels fit with the projections of the first substrate; and a third substrate having a plurality of channels corresponding with the top surfaces of the projections of the first substrate and a plurality of channels corresponding with the substrate surface between the channels of the second substrate.

The first substrate may further comprise at least one fixing pillar and the second substrate and the third substrate each may further comprise at least one fixing channel into which the at least one fixing pillar can be inserted.

The thickness of the second substrate may be the same as the height of the projections of the first substrate. However, the thickness of the second substrate may be different from the height of the projections of the first substrate, provided that after cells are seeded on the first substrate and a different type of cells are seeded on the second substrate, the interaction between the different types of cells can be observed during the co-culture of the cells.

The first substrate, except the projections of the first substrate, may be formed of glass, silicone, plastics, or a combination thereof, but is not limited thereto.

The projections of the first substrate, the second substrate, and the third substrate may be formed of a biocompatible material, preferably SU-8.

In the cell co-culture apparatus, the width, length, and height of the projections of the first substrate may each be from 50 to 100 µm, the width and length of the channels of the second substrate and the thickness of the second substrate may each be from 50 to 100 µm, and the width and length of the channels of the third substrate and the thickness of the third substrate may each be from 50 to 100 µm. The above mentioned dimensions of the width, length and height/thickness of the projections, the second substrate and the third substrate may be the same or different.

According to another aspect of the present invention, there is provided a method of co-culturing and isolating cells using the cell co-culture apparatus, comprising: disposing a second substrate on a first substrate such that a plurality of channels of the second substrate fit with a plurality of projections of the first substrate, respectively, and then disposing a third substrate on the second substrate such that a plurality of channels of the third substrate correspond with either the top surfaces of the projections of the first substrate or the substrate surface between the channels of the second substrate, respectively; seeding first cells on the top surfaces of the projections of the first substrate and second cells on the substrate surface between the channels of the second substrate through the channels of the third substrate; separating the third substrate from the second substrate and culturing the cells; and separating the second substrate from the first substrate.

In the method, the same or different type of the first cells may be seeded on each of the top surfaces of the projections of the first substrate. Also, the same or different type of the second cells may be seeded on each of the substrate surface between the channels of the second substrate.

According to another aspect of the present invention, there is provided a method of co-culturing and isolating cells using two cell co-culture apparatus, the method comprising: disposing a second substrate on a first substrate such that a plurality of channels of the second substrate fit with a plurality of projections of the first substrate, respectively, and disposing a third substrate on the second substrate such that a plurality of channels of the third substrate correspond with either the top surfaces of the projections of the first substrate or the substrate surface between the channels of the second substrate, respectively, for each set of the substrates of the two cell co-culture apparatuses according to an embodiment of the present invention, seeding first cells through all the channels of the third substrate in the first cell co-culture apparatus, seeding second cells through all the channels of the third substrate in the second cell co-culture apparatus, and separating the first substrate, the second substrate, and the third substrate from each other, for each of the first and second cell co-culture apparatuses; disposing the second substrate of the second cell co-culture apparatus on the first substrate of the first cell co-culture apparatus and the second substrate of the first cell co-culture apparatus on the first substrate of the second cell co-culture apparatus such that the channels of the second substrate fit with the projections of the first substrate, respectively, and then culturing the cells; and separating the second substrates from the first substrates, respectively.

### BRIEF DESCRIPTION OF THE DRAWINGS

The above and other features and advantages of the present invention will become more apparent by describing in detail exemplary embodiments thereof with reference to the attached drawings in which:
FIG. 1A is a schematic view illustrating a hematopoietic stem cell (HSC) in a resting phase adjacent to spindle-shaped N-cadherin osteoblast (SNO) in humans;
FIG. 1 B is a schematic view illustrating an interaction between the HSC and the SNO via N-cadherin in order for the HSC to retain stemness;
FIG. 2 is a schematic view illustrating a conventional transwell insert used in researching an interaction between cells by a soluble factor through a liquid;
FIG. 3 shows schematic perspective views illustrating a cell co-culture apparatus for researching the interaction between cells according to an embodiment of the present invention;
FIG. 4A is a schematic perspective view illustrating a first substrate of a cell co-culture apparatus for researching the interaction between cells according to an embodiment of the present invention;
FIG. 4B is a schematic perspective view illustrating a first substrate of a cell co-culture apparatus for researching the interaction between cells according to another embodiment of the present invention;
FIG. 5 is a schematic perspective view illustrating a second substrate of a cell co-culture apparatus for researching the interaction between cells according to an embodiment of the present invention;
FIG. 6 is a schematic perspective view illustrating a third substrate of a cell co-culture apparatus for researching the interaction between cells according to an embodiment of the present invention;
FIG. 7 is a schematic perspective view illustrating a method of sequentially disposing a second substrate and a third substrate on a first substrate of a cell co-culture apparatus according to an embodiment of the present invention, wherein the first substrate further comprises a plurality of fixing pillars and the second substrate and the third substrate further comprise a plurality of fixing channels into which the fixing pillars are inserted;
FIG. 8 is a schematic perspective view illustrating a stacked form of the cell co-culture apparatus according to an embodiment of the present invention in which the second substrate is disposed on the first substrate;
FIG. 9 is a schematic perspective view illustrating a stacked form of the cell co-culture apparatus according to an embodiment of the present invention in which the second substrate is disposed on the first substrate, and then the third substrate is disposed on the second substrate;
FIG. 10 illustrates a method of co-culturing and isolating cells according to an embodiment of the present invention using the cell co-culture apparatus according to an embodiment of the present invention;
FIG. 11 illustrates another method of co-culturing and isolating cells according to another embodiment of the present invention using the cell co-culture apparatus according to another embodiment of the present invention.
FIG. 12 illustrates microscopic photographs of cells inoculated in a cell co-culture apparatus according to an embodiment of the present invention taken after a third substrate is separated from the apparatus; and
FIG. 13 illustrates microscopic photographs of cells on a first substrate and a second substrate co-cultured in a cell co-culture apparatus according to an embodiment of the present invention, the photographs taken after the first and second substrates are separated from each other.

### DETAILED DESCRIPTION OF THE INVENTION

Hereinafter, the present invention will be described in more detail with reference to the attached drawings. The purposes and advantages of the present invention will become more apparent by describing the following explanation. However, exemplary embodiments illustrated in the attached drawings may be modified into other various forms, and it should not be interpreted that the scope of the present invention is limited to the exemplary embodiments illustrated in the attached drawings. The exemplary embodiments of the present invention are provided for the purpose of explaining the invention more completely to those of ordinary skill in the art. Throughout the specification, identical reference numerals denote identical elements. Further, various elements and regions are roughly illustrated in the drawings. Thus, the present invention is not limited by relative sizes or intervals illustrated in the attached drawings.

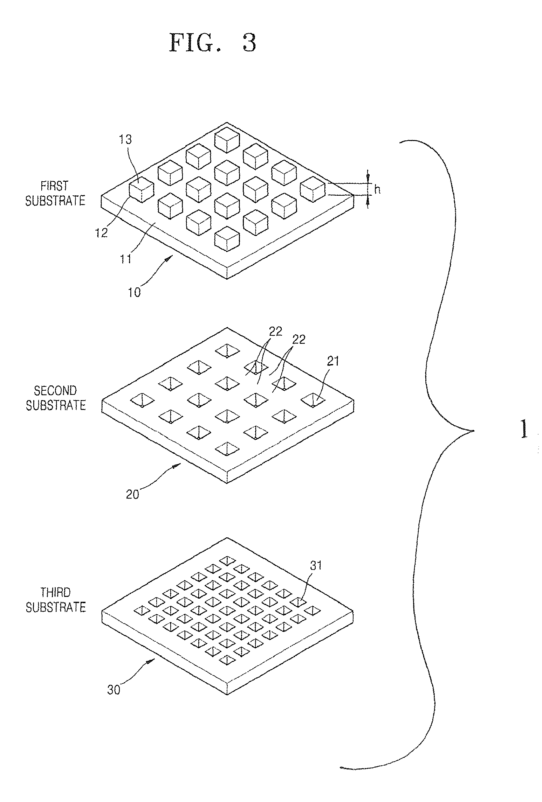

FIG. 3 illustrates a cell co-culture apparatus 1 for researching an interaction between cells according to an embodiment of the present invention.

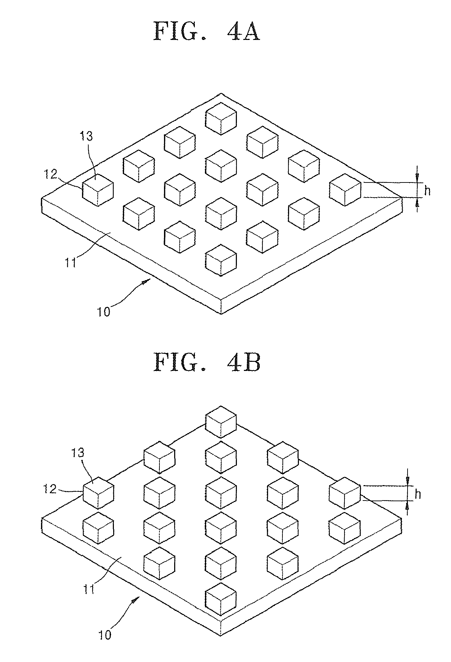

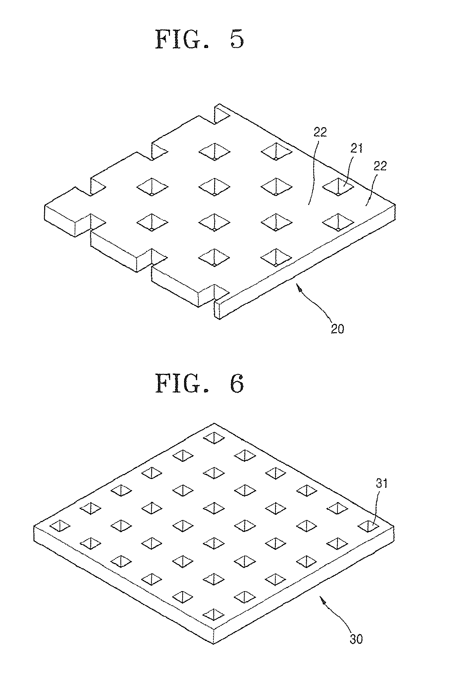

Referring to FIG. 3, the cell co-culture apparatus 1 comprises a first substrate 10, a second substrate 20, and a third substrate 30. Exemplary embodiments of the first substrate 10 are illustrated in FIGS. 4A and 4B and exemplary embodiments of the second substrate 20 and the third substrate 30 are illustrated in FIGS. 5 and 6, respectively.

In the cell co-culture apparatus 1, the first substrate 10, the second substrate 20, and the third substrate 30 are sequentially disposed, and cells are seeded through the channels 31 of the third substrate 30. The third substrate 30 is separated from the second substrate 20, and then the cells are cultured. The cell co-culture apparatus 1 consists of the three substrates 10, 20 and 30.

The first substrate 10 has a plurality of projections 12 on a surface 11 of the first substrate 10, wherein the projections 12 have flat top surfaces 13 for culturing cells. The flat top surfaces 13 of the projections 12 are portions on which the cells are seeded and cultured. Thus, the projections 12 should have sufficient flat top surfaces 13 such that the cells can be seeded and cultured. The projections 12 may have any one of various shapes, for example, a cylinder or a polygonal pillar, including a cube. The height of the projections 12 is not specifically limited. That is, if the height of the projections 12 is such that a difference between the height of the projections 12 and the thickness of the second substrate 20 allows an interaction to occur between the cells seeded on the substrate surface 22 (the substrate surface 22 being those surface regions of the second substrate that lie between the channels 21) and the cells seeded on the flat top surfaces 13 during the co-culture, then the height of the projections 12 may be adjusted to a reasonable level. The number of the projections 12 of the first substrate 10 is not specifically limited, and may depend on the intended number of portions in which the cells are to be seeded during the co-culture. The projections 12 may have an arrangement as illustrated in FIG. 4A or FIG. 4B, but the arrangement of the projections 12 is not limited thereto.

FIG. 5 illustrates the second substrate 20, which is included in a cell co-culture apparatus 1 for researching the cell interaction according to an embodiment of the present invention. The second substrate 20 illustrated in FIG. 5 may be coupled with the first substrate 10 illustrated in FIG. 4B.

The second substrate 20 is a substrate for seeding and culturing the second cells from among the cells that are to be investigated for cell interaction, and has the same number of channels 21 as the number of projections 12 of the first substrate 10 illustrated in FIG. 4B. The channels 21 fit with the projections 12 of the first substrate 10. The cells are seeded and cultured on the substrate surface 22 between the channels 21 of the second substrate 20. Thus, the channels 21 of the second substrate 20 must have a shape that corresponds with the projections 12 of the first substrate 10. The shapes and positions of the channels 21 of the second substrate 20 will depend on the shapes and positions of the projections 12 of the first substrate 10.

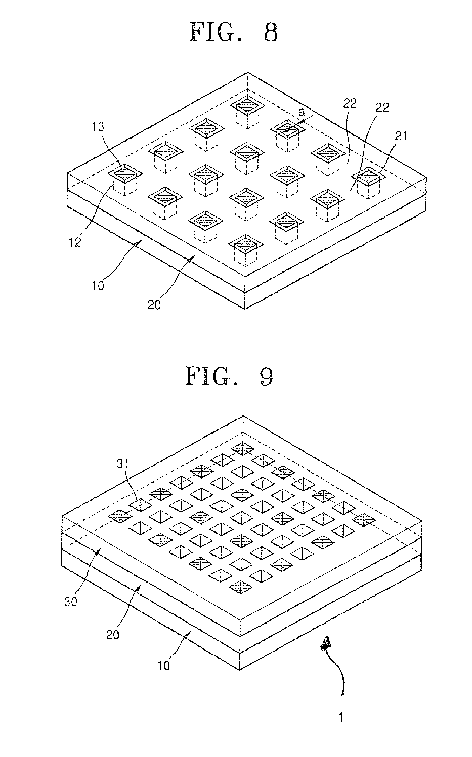

The size of the channels 21 of the second substrate 20 may be the same as the size of the cross-sections of the projections 12. However, when the second substrate 20 is disposed on the first substrate 10, an interval "a" between the flat top surfaces 13 of the projections 12 of the first substrate 10 and the substrate surface 22 of the second substrate 20 (see FIG. 8) may be present, the interval "a" being so narrow that the cells on the flat top surfaces 13 of the projections 12 of the first substrate 10 can interact with the cells on the substrate surface 22. The interval "a" depends on the types of co-cultured cells. For example, the interval "a" is 10 to 20 µm.

Furthermore, the interval "a" may be adjusted in various ways in order to confirm an effect of the distance between the cells that is to be investigated during the interaction between the cells.

The thickness of the second substrate 20 may be the same as the height "h" of the projections 12 of the first substrate 10 so as to allow the interaction between the cells to be cultured on the flat top surfaces 13 of the projections 12 of the first substrate 10 and the cells to be cultured on the substrate surface 22 between the channels 21 of the second substrate 20. However, the thickness of the second substrate 20 may be different from the height "h" of the projections 12 of the first substrate 10 so that such a difference allows the interaction between the cells. The difference is very small and depends on the types of co-cultured cells, for example, 10 to 20 µm.

FIG. 6 illustrates the third substrate 30 that is included in a cell co-culture apparatus 1 for researching the interaction between cells according to an embodiment of the present invention. The third substrate 30 illustrated in FIG. 6 may be coupled with the first substrate 10 illustrated in FIG. 48 and the second substrate 20 illustrated in FIG. 5.

The third substrate 30 has a plurality of channels 31 through which the cells may easily be seeded on the flat top surfaces 13 of the projections 12 of the first substrate 10 and the substrate surface 22 between the channels 21 of the second substrate 20. Thus, the third substrate 30 has a plurality of channels 31 corresponding with the flat top surfaces 13 of the projections 12 of the first substrate 10 and a plurality of channels 31 corresponding with the substrate surface 22 between the channels 21 of the second substrate 20. As a result, an arrangement form of the channels 31 of the third substrate 30 will depend on the positions of the projections 12 of the first substrate 10 and the substrate surface 22 between the channels 21 of the second substrate 20. Even if, referring to FIG. 3, a cross-section of the channels 31 of the third substrate 30 is rectangular, the cross-section may be in any shape that allows the introduction of the cells into the channels 31, for example, a circle or a polygon. The thickness of the third substrate 30 is any thickness that allows an easy introduction of the cells, and may be preferably 50to 100 µm.

In the cell co-culture apparatus 1 according to an embodiment of the present invention, the width, length, and height of the projections 12 of the first substrate 10 may each be from 50 to 100 µm; the width and length of the channels 21 of the second substrate 20 and the thickness of the second substrate 20 may each be from 50 to 140 µm; and the width and length of the channels 31 of the third substrate 30 and the thickness of the third substrate 30 may each be from 50 to 100 µm, but are not limited thereto. Preferably, the width, length, and height of the projections 12 of the first substrate 10 may be 0.1 x 0.1 x 0.1 mm; the width and length of the channels 21 of the second substrate 20 may be 0.14 x 0.14 mm and the thickness of the second substrate 20 may be 0.1 mm; and the width and length of the channels 31 of the third substrate 30 may be 0.09 x 0.09 mm and the thickness of the third substrate 30 may be 0.1 mm.

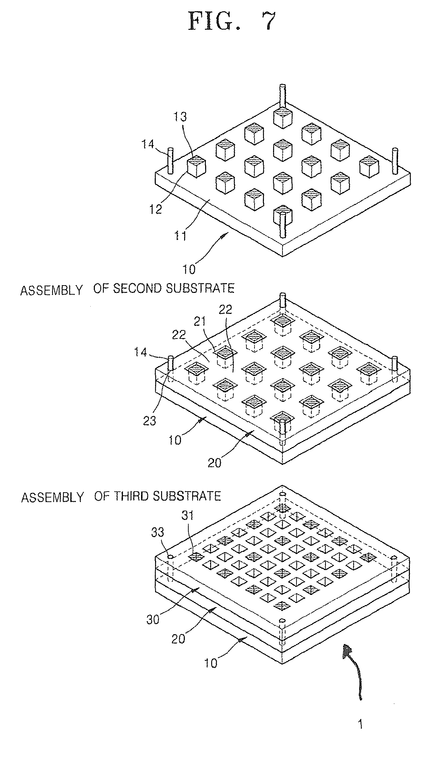

FIG. 7 is a schematic perspective view illustrating a method of sequentially disposing a second substrate 20 and a third substrate 30 on a first substrate 10 of a cell co-culture apparatus 1 according to an embodiment of the present invention, the first substrate 10 further comprising a plurality of fixing pillars 14 and the second substrate 20 and the third substrate 30 further comprising a plurality of fixing channels 22 and 33 into which the fixing pillars 14 are inserted. In the cell co-culture apparatus 1, the first substrate 10 further comprises the plurality of fixing pillars 14 on the first substrate surface 11 such that the second substrate 20 and the third substrate 30 can be more stably disposed on the first substrate 10. The second substrate 20 and the third substrate 30 further comprise the fixing channels 23 and 33 into which the fixing pillars 14 on a first substrate surface 11 are inserted.

Since the fixing pillars 14 on the first substrate 10 are intended to stably fix with the second substrate 20 and the third substrate 30, the number of the fixing pillars 14 is not specifically limited and the fixing pillars 14 may be present in any positions as long as the positions of the fixing pillars 14 do not have an effect on the interaction between the cells. The fixing pillars 14 may be in any pillar shape, including a cylinder and a polygonal pillar, provided that the fixing channels 23 and 33 correspond to such forms and fit with the corresponding fixing pillars 14. Referring to FIG. 7, the first substrate 10 has one fixing pillar 14 in the shape of a cylinder in each of the corners of the first substrate 10, and the second substrate 20 and the third substrate 30 have the fixing channels 23 and 33 in corresponding corners such that the fixing channels 23 and 33 can correspondingly fit with the fixing pillars 14.

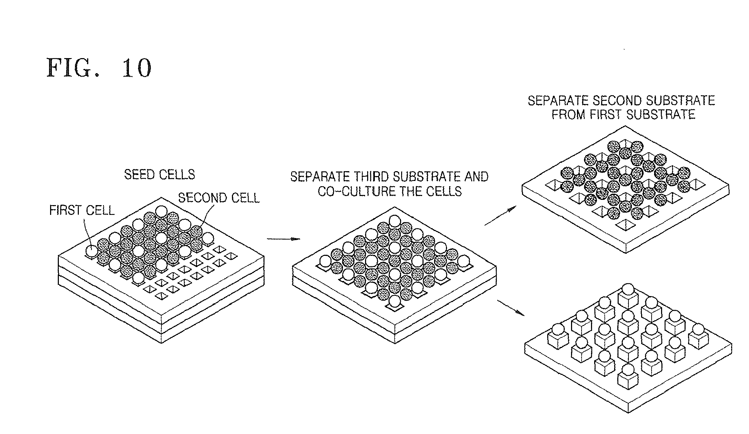

The cell co-culture apparatus 1 may be used to confirm what interaction has occurred between the different types of cells by co-culturing the cells and separating a type of cells from the other type of cells. FIG. 10 illustrates a method of co-culturing and isolating cells using the cell co-culture apparatus 1, illustrated in FIG. 8, according to an embodiment of the present invention.

To research the interaction between the cells using the cell co-culture apparatus 1, referring to FIG. 8, at first, the second substrate 20 is disposed on the first substrate 10. That is, the second substrate 20 is disposed on the first substrate 10 such that the channels 21 of the second substrate 20 fit with the projections 12 of the first substrate 10, respectively. In this case, there may be a difference between the height "h" of the projections 12 of the first substrate 10 and the thickness of the second substrate 20. However, the height "h" of the projections 12 of the first substrate 10 may advantageously be the same as the thickness of the second substrate 20 such that the flat top surfaces 13 of the projections 12 of the first substrate 10 and the substrate surface 22 of the second substrate 20 form a flat surface after the assembly.

Subsequently, the third substrate 30 is disposed on the second substrate 20 such that a plurality of channels 31 of the third substrate 30 corresponds with the flat top surfaces 13 of the projections 12 of the first substrate 10 and the substrate surface 22 between the channels 21 of the second substrate 20, respectively. FIG. 9 is a schematic perspective view illustrating a stacked form of the cell co-culture apparatus 1 formed by disposing the first substrate 10, the second substrate 20, and the third substrate 30 sequentially, according to an embodiment of the present invention. After the third substrate 30 is disposed on the second substrate 20, the first cells are seeded on the flat top surfaces 13 of the projections 12 of the first substrate 10 and the second cells are seeded on the substrate surface 22 between the channels 21 of the second substrate 20 through the channels 31 of the third substrate 30. Since the first cells are different from the second cells and the first cells and the second cells may be cultured on the flat top surfaces 13 of the projections 12 of the first substrate 10 and the substrate surface 22 between the channels 21 of the second substrate 20, respectively, the interaction between the cells by direct contact may occur.

All the first cells that are seeded on the flat top surfaces 13 of the projections 12 of the first substrate 10 may be identical. However, to simultaneously research the interactions between the various combinations of cells, different types of cells may be seeded on the flat top surfaces 13 of the projections 12 of the first substrate 10 through each of the channels 31 of the third substrate 30.

All the second cells that are seeded on the substrate surface 22 between the channels 21 of the second substrate 20 may be identical. However, to simultaneously research the interactions between the various combinations of cells, different types of cells may be seeded on the substrate surface 22 between the channels 21 of the second substrate 20 through each of the channels 31 of the third substrate 30.

After a desired combination of cells are seeded on the flat top surfaces 13 of the projections 12 of the first substrate 10 and the substrate surface 22 through the channels 31 of the third substrate 30, the third substrate 30 is separated from the second substrate 20 and then, the cells are co-cultured. The cell co-culture allows direct contact between the cells on the flat top surfaces 13 and the cells on the substrate surface 22 to sufficiently occur after the separation of the third substrate 30.

When the cell co-culture is completed, the second substrate 20 is separated from the first substrate 10. Then, the cells are taken and directly observed in order to confirm if a change occurred in each of the first cells and the second cells. Due to the easy separation of the first substrate 10 from the second substrate 20, it can be confirmed which change occurred in each of the first cells and the second cells due to the direct contact between the different cells.

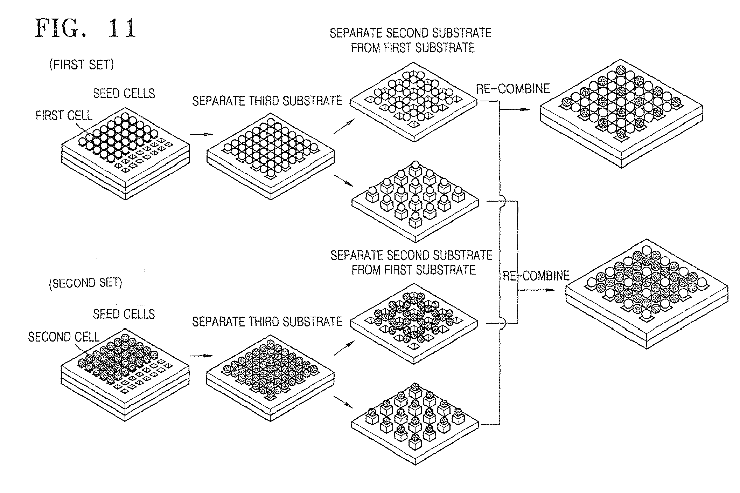

The cell co-culture apparatus 1 may be used in different ways. FIG. 11 illustrates a method of using the cell co-culture apparatus 1 according to another embodiment of the present invention.

At first, two cell co-culture apparatuses 1 according to an embodiment of the present invention are prepared. For each set of the substrates of both cell co-culture apparatuses 1, a second substrate 20 is disposed on a first substrate 10 such that a plurality of channels 21 of the second substrate 20 fit with a plurality of projections 12 of the first substrate 10, respectively. Then, a third substrate 30 is disposed on the second substrate 20 such that a plurality of channels 31 of the third substrate 30 correspond with the flat top surfaces 13 of the projections 12 of the first substrate 10 and substrate surface 22 between the channels 21 of the second substrate 20, respectively. Subsequently, the first cells are seeded through all the channels 31 of the third substrate 30 in the first cell co-culture apparatus 1, and the second cells are seeded through all the channels 31 of the third substrate 30 in the second cell co-culture apparatus1. Then, the first substrate 10, the second substrate 20, and the third substrate 30 in each of the first and second cell co-culture apparatuses 1 are separated from each other. As illustrated in FIG. 11, the first and second substrates 10 and 20 of the first cell co-culture apparatus 1 and the first and second substrates 10 and 20 of the second cell co-culture apparatus 1 are alternately disposed. That is, the second substrate 20 of the second cell co-culture apparatus 1 is disposed on the first substrate 10 of the first cell co-culture apparatus 1 and the second substrate 20 of the first cell co-culture apparatus 1 is disposed on the first substrate 10 of the second cell co-culture apparatus 1 such that the channels 21 of the second substrate 20 fit with the projections 12 of the first substrate 10, respectively, and then cell culture is performed on each set of substrates. When the cell culture is completed, the second substrate 20 is separated from the first substrate 10 and the results of the changes that occurred in each cell may be confirmed.

Hereinafter, the present invention will be described in more detail with reference to the following examples. However, these examples are given for the purpose of illustration and are not intended to limit the scope of the invention.

### EXAMPLES

### Example 1: Co-culture of cells

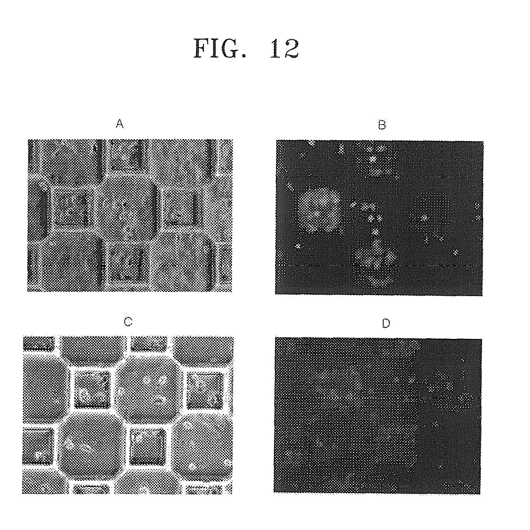

A cell co-culture apparatus 1 was prepared using a first substrate 10, a second substrate 20, and a third substrate 30, respectively, as illustrated in FIGS. 4B, 5 and 6. The base of the first substrate 10, but not the projections 12 of the first substrate 10, was formed of glass, and the projections 12 of the first substrate 10, the second substrate 20, and the third substrate 30 were formed of SU-8 (Microchem). The width, length, and height of the projections 12 of the first substrate 10 were 50 µm. The width and length of the channels 21 of the second substrate 20 and the thickness of the second substrate 20 were 50 µm. The width and length of the channels 31 of the third substrate 30 and the thickness of the third substrate 30 were 50 µm. The first substrate 10, the second substrate 20, and the third substrate 30 were sequentially disposed, and then, A549 cells, which are human cells, were inoculated on the apparatus 1 through the channels 31 of the third substrate 30. For each of the channels 31 of the third substrate 30, two to five cells were inoculated. Subsequently, the third substrate 30 was separated from the second substrate 20, and then the flat top surfaces 13 of the first substrate 10 and the substrate surface 22 of the second substrate 20 on which the cells were inoculated were photographed using a phase-contrast microscope. The photographs are indicated as A and C in FIG. 12.

To confirm whether the cells observed on the above substrates are alive, the cells were fluorescence-stained with CellTracker™ Green CMFDA (5-Chloromethylfluorescein Diacetate) or CellTracker™ Red (Invitrogen), and photographed using a fluorescence microscope. The photographs are indicated as B and D in FIG. 12. FIG. 12B illustrates the cells stained with CellTracker™ Green CMFDA (Invitrogen) emitting green fluorescence and FIG. 12D shows the cells stained with CellTracker™ Red CMTPX (Invitrogen) emitting red fluorescence.

Referring to A through D of FIG. 12, it can be confirmed that the cells are uniformly distributed on the top surfaces 13 of the projections 12 of the first substrate 10 and on the substrate surface 22 between the channels 21 of the second substrate 20.

### Example 2: Isolation of co-cultured cells

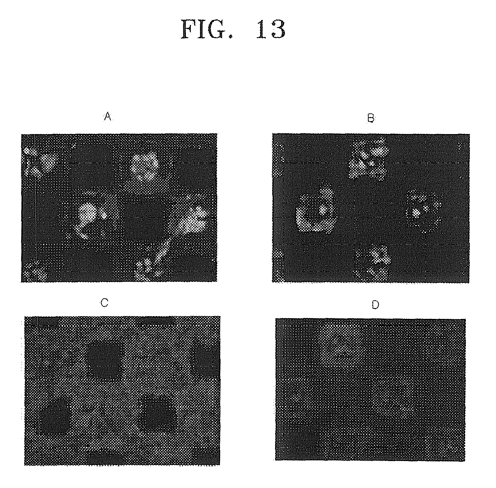

The cells that were inoculated in Example 1 were cultured at 37°C for 24 hours after the separation of the third substrate 30 from the second substrate 20. Then, the first substrate 10 and the second substrate 20 were separated from each other. The cells on the first substrate 10 and the cells on the second substrate 20 were fluorescence-stained with CellTracker™ Green CMFDA (Invitrogen) or CellTracker™ Red CMTPX (Invitrogen), and photographed using a fluorescence microscope. The photographs are illustrated in FIG. 13. A and B in FIG. 13 illustrate the cells stained with CellTracker™ Green CMFDA (Invitrogen) emitting green fluorescence. C and D in FIG. 13 illustrate the cells stained with CellTracker™ Red CMTPX (Invitrogen) emitting red fluorescence.

A and C in FIG. 13 represent the cells cultured on the substrate surface 22 between the channels 21 of the second substrate 20. It can be confirmed from A and C that the cells cultured on the projections 12 of the first substrate 10 (B and D) were separated from the cells on the substrate surface 22 of the second substrate 20. Meanwhile, B and D in FIG. 13 represent the cells cultured on the flat top surfaces 13 of the projections 12 of the first substrate 10. In B and D in FIG. 13, the cells cultured on portions other than the projections 12 of the first substrate 10, i.e., the cells cultured on the portions of the second substrate 20 (A and C), were removed.

It can be confirmed from the above results that according to an embodiment of the present invention, a desired pattern of cell culture can be made, and the cells co-cultured on a combination of the first substrate 10 and the second substrate 20 can be isolated in a desired pattern by separating the first substrate 10 and the second substrate 20 from each other.

As described above, according to the present invention, a cell co-culture apparatus 1 for researching the interaction between the cells by direct contact may be provided. Further, a method of co-culturing and isolating cells for researching the interaction between the cells by direct contact may be provided using the cell co-culture apparatus 1.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A cell co-culture apparatus (1) comprising:
a first substrate (10) having a plurality of projections (12) on the surface (11) of the first substrate (10), wherein the projections (12) have top surfaces (13) for culturing cells;
a second substrate (20) having the same number of channels (21) as the number of the projections (12) of the first substrate (10) such that said channels (21) fit with the projections (12) of the first substrate (10); and
a third substrate (30) having a plurality of channels (31) corresponding with either the top surfaces (13) of the projections (12) of the first substrate (10) or with the substrate surface (22) between the channels (21) of the second substrate (20).

2. The cell co-culture apparatus (1) of claim 1, wherein the first substrate (10) further comprises at least one fixing pillar (14) and the second substrate (20) and the third substrate (30) each further comprise at least one fixing channel (23) into which the at least one fixing pillar (14) can be inserted.

3. The cell co-culture apparatus (1) of claim 1, wherein the thickness of the second substrate (20) is the same as the height of the projections (12) of the first substrate (10).

4. The cell co-culture apparatus (1) of claim 1, wherein the first substrate (10), except the projections, is formed of glass, silicone, plastics, or a combination thereof.

5. The cell co-culture apparatus (1) of claim 1, wherein the projections (12) of the first substrate (10), the second substrate (20), and the third substrate (30) are formed of a biocompatible material.

6. The cell co-culture apparatus (1) of claim 5, wherein the biocompatible material is SU-8.

7. The cell co-culture apparatus (1) of claim 1, wherein the width, length, and height of the projections (12) of the first substrate (10) are each from 50 to 100 µm,
the width and length of the channels (21) of the second substrate (20) and the thickness of the second substrate (20) are each from 50 to 100 µm, and
the width and length of the channels of the third substrate (30) and the thickness of the third substrate (30) are each from 50 to 100 µm.

8. A method of co-culturing and isolating cells using the cell co-culture apparatus (1) of any one of claims 1 to 7, comprising:
disposing a second substrate (20) on a first substrate (10) such that a plurality of channels (21) of the second substrate (20) fit with a plurality of projections (12) of the first substrate (10), respectively, and then disposing a third substrate (30) on the second substrate (20) such that a plurality of channels (31) of the third substrate (30) corresponds with either the top surfaces (13) of the projections (12) of the first substrate (10) or the substrate surface (22) between the channels (21) of the second substrate(20), respectively;
seeding first cells on the top surfaces (13) of the projections (12) of the first substrate (10) and second cells on the substrate surface (22) between the channels (21) of the second substrate (20) through the channels (31) of the third substrate (30);
separating the third substrate (30) from the second substrate (20) and culturing the cells; and
separating the second substrate (20) from the first substrate (10).

9. The method of claim 8, wherein the same or different type of first cells are seeded on each of the top surfaces (13) of the projections (12) of the first substrate (10).

10. The method of claim 8, wherein the same or different type of second cells are seeded on each of the substrate surface (22) between the channels (of the second substrate (20).

11. A method of co-culturing and isolating cells using two cell co-culture apparatuses (1) of any one of claims 1 to 7, comprising:
disposing a second substrate (20) on a first substrate (10) such that a plurality of channels (21) of the second substrate (20) fit with a plurality of projections (12) of the first substrate (10), respectively, and disposing a third substrate (30) on the second substrate (20) such that a plurality of channels (31) of the third substrate (30) correspondswith either the top surfaces (13) of the projections (12) of the first substrate (10) or the substrate surface (22) between the channels (21) of the second substrate (20), respectively, for each set of substrates of the two cell co-culture apparatuses (1) of any one of claims 1 to 7,
seeding first cells through all the channels (31) of the third substrate (30) in the first cell co-culture apparatus (1), seeding second cells through all the channels of the third substrate (30) in the second cell co-culture apparatus (1), and separating the first substrate (10), the second substrate (20), and the third substrate (30) from each other, for each of the first and second cell co-culture apparatuses (1);
disposing the second substrate (20) of the second cell co-culture apparatus (1) on the first substrate (10) of the first cell co-culture apparatus (1) and the second substrate (20) of the first cell co-culture apparatus (1) on the first substrate (10) of the second cell co-culture apparatus (1) such that the channels of the second substrate (20) fit with the projections (12) of the first substrate (10), respectively, and then culturing the cells; and
separating the second substrates (20) from the first substrates (10), respectively.
